(19) Europäisches Patentamt / European Patent Office / Office européen des brevets

(11) **EP 4 617 357 A1**

(12) **EUROPEAN PATENT APPLICATION**

(43) Date of publication:
**17.09.2025 Bulletin 2025/38**

(21) Application number: **24305374.1**

(22) Date of filing: **12.03.2024**

(51) International Patent Classification (IPC):
*C12M 1/42* (2006.01)  *C12M 1/00* (2006.01)

(52) Cooperative Patent Classification (CPC):
**C12M 35/04; C12M 47/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Aenitis Technologies**
**77290 Mitry-Mory (FR)**

(72) Inventors:
• **BERTIN, Nicolas**
**77290 Mitry-Mory (FR)**
• **GORJI, Azita**
**77290 Mitry-Mory (FR)**
• **BAUDOUIN, Vincent**
**77290 Mitry-Mory (FR)**
• **VINCENT, Emmannuel**
**77290 Mitry-Mory (FR)**

(74) Representative: **Icosa**
**83 avenue Denfert-Rochereau**
**75014 Paris (FR)**

(54) **ACOUSTIC CELL SEGREGATION METHOD AND SYSTEM**

(57)    The present invention relates to a method for segregating cells (2) and an acoustophoresis device (1) for implementing said method.

**FIG. 5**

## Description

### FIELD OF INVENTION

The present invention relates to segregating components in a cell suspension, and more particularly segregating cells in suspension into a fluid.

### BACKGROUND OF INVENTION

Various methods have been developed to achieve cell isolation and segregation, each with its unique strengths and limitations. Known techniques such as: Immunomagnetic Cell Separation (ICS), Fluorescence-activated Cell Sorting (FACS), rely on microfluidic devices which provides precise control over fluid flow at the microscale. However, FACS method needs the targeted cells to be fluorescently labelled and this fluorescent state remains even when the method and the segregation is finished. Other drawbacks are inherent to the FACS method, such as the known marker limitation for targeted cells, cell stress induced by the method and the limitation to homogeneous samples of cells. The Density Gradient Centrifugation (DGC) method is also known and allows a label free cell sorting. However, the process is time consuming and may cause cell activation. Immunodensity Cell Separation is a method that combines both antibody targeting and gradient centrifugation, using density gradients to separate cells based on their buoyant densities. However, ICS is limited only to negative detection. Mechanical or gravitational methods such as sedimentation, centrifugation, adhesion, microfluidic cell separation or filtration also have several drawbacks: potential damage to the cells, lack of specificity, small sample processing, time consuming and/or scalability limitation. Magnetic-activated cell sorting (MACS) rely on magnetic nanoparticles associated to specific antibodies to selectively label and isolate target cells, providing a label-based approach for cell separation. However, MACS is limited in terms of purity and high throughput application. Furthermore, as magnetic nanoparticles are not good candidates for injection in a subject as they would have to be eliminated by the kidneys, meaning that before any injection in a subject, collected cells must be washed to eliminate the magnetic nanoparticles, leading to a time-consuming method and potential damage to the collected cells.

To conclude, all these methods often suffer from low throughput, limited scalability, potential loss of cells hindering the segregation yield, and potential cell damage.

Thus, there is a need for a cell segregation method showing high cell specificity coupled to high segregation efficiency while being easily scalable and damage free.

The Applicant has found that this need could be met with a method using acoustic waves coupled with attachment of the cells to a differentiation vector.

## SUMMARY

The present invention relates to a method for segregating cells in a suspension comprising:

(S1). providing an acoustophoresis device comprising:

i. a chamber configured to be associated with at least one acoustic wave generator for generating acoustic waves within the chamber;

ii. at least one acoustic wave generator configured to generate acoustic waves within the chamber;

iii. at least one inlet in fluid communication with the chamber;

iv. at least three outlets in fluid communication with the chamber, said outlets comprising a first central outlet for evacuation of a first part of the suspension and at least two second peripheral outlets for evacuation of a second part of the suspension;

(S2). preparing a suspension comprising cells of interest in a medium, wherein cells of interest are attached to a differentiation vector,

(S3). injecting the suspension comprising the cells of interest into the chamber at the inlet;

(S4). applying an acoustic field by generating acoustic waves inside the chamber;

(S5). collecting the cells of interest attached to the differentiation vector throughout the at least two second peripheral outlets.

In the present disclosure the medium may be any type of fluid, for instance a buffer medium, water, biological buffer medium, or a mixture thereof. "Buffer" refers to a fluid in which deflected target particles will be deviated when the device is activated. The buffer may be functionalized through the addition of chemical compounds and/or biological vectors, considered as particles, such as viruses, bacteria, DNA (Deoxyribonucleic acid), RNA (Ribonucleic acid), plasmids, proteins designed for example to react with or transfect deflected target particles. The buffer may be CPD (Citrate, Phosphate, Dextrose), Platelet additive solution, RPMI (Roswell Park Memorial Institute medium) medium, LB (lysogeny broth), HypoThermoSol®, UW (University of Wisconsin) solution, HEPES (4-(2-hydroxyethyl)-1-piperazineethanesulfonic acid), PBS (phosphate buffered saline), CMRL medium or DMEM (Dulbecco's Modified Eagle medium), MEM (Minimum Essential Medium), Ham's F12 (F-12 Nutrient Medium), IMDM (Iscove's Modified Dulbecco's Medium), DMEM/F12 (Hybride DMEM and F12)

The method is particularly adapted to suspension and medium being a biological fluid selected in the group comprising human and/or non-human cell suspension, cell cluster suspension, blood, whole blood, surgical blood, platelet rich plasma, buffy coat, urine, serum, lymph, fluidified feces, adipose tissue, bone marrow, cerebrospinal fluid, sperm, cord blood, milk, saliva, tissue, egg albumen, seashell mix, or a mixture thereof. Targeted cells may be selected in the group comprising, but not limited to: stem cells such as for example differentiated stem cells, undifferentiated stem cells, IPS (Induced pluripotent stem cells), mesenchymal stem cells, culture cells, megakaryocytes, cell clusters such as for example Langerhans islets, micro-alga, bacteria, organoids, cells lines, yeasts.

Acoustophoresis has emerged as a promising technique for label-free, contactless manipulation of cells within microfluidic systems. By harnessing acoustic forces generated by ultrasonic waves, acoustophoresis enables the precise separation of cells based on their mechanical properties, including size, density, and compressibility. In this patent, we propose a novel method for cell isolation using droplets or bubbles in conjunction with acoustophoresis. By coupling cells with droplets or bubbles suspended in a continuous phase, we aim to enhance the efficiency, throughput, and purity of cell isolation while minimizing cell damage and sample loss. This innovative approach has the potential to revolutionize cell sorting and isolation, offering new opportunities for biological research, clinical diagnostics, and therapeutic interventions.

Advantageously, this method renders possible to efficiently separate the targeted cells of interest that are attached to a differentiation vector from the other cells. In other words, according to this method, it becomes possible to separate, for instance, rare cells from a suspension of cells. The segregation may be either positive (i.e. cells of interest are to be kept), or negative (i.e. cells of interest are to be separated from the other cells).

Indeed, the differentiation vector allows modifying the properties of the targeted cell to which it is attached. For instance, if targeted cells have a density and compressibility identical or similar to the density and compressibility of the suspension medium or other cells comprised in the suspension, it will be difficult to segregate said targeted cells using acoustic means. The differentiation vector may have a different density and compressibility than the suspension medium and the targeted cell, allowing to render the targeted cell density distinctive from the suspension medium and other cells comprised in the suspension. Thus, since targeted cells which are attached to a differentiation vector have modified properties, advantageously modified density, the acoustic field generated into the acoustophoresis device allows separating the cells attached to a differentiation vector from the other cells present in the suspension.

On the other hand, coupling the use of a differentiation vector to an acoustophoresis device is also advantageous because segregating cells using acoustic forces prevents damaging the cells. Thus, the method of the invention allows keeping the advantages of acoustophoresis over filtration or centrifugation.

The acoustic wave generator may be configured to generate bulk acoustic waves (BAW), i.e. volume acoustic waves propagating in 3 dimensions. Indeed, the acoustic wave generator 14 does not generate surface acoustic waves and does not relate on gravity effect to segregate cells from a suspension. This allows advantageously for bigger volumes to be treated.

In some embodiments, the second part of the suspension comprises cells of interest, while the first part of the suspension is depleted of cells of interest. This embodiment corresponds to a positive segregation. On the other hand, the first part of the suspension may comprise cells of interest, while the second part of the suspension may be depleted of cells of interest. This embodiment corresponds to a negative segregation.

In some embodiments, the differentiation vector comprises:

(S1). an anchor configured to be attached to a cell of interest and

(S2). a low-density body attached to the anchor,

wherein the low-density body comprises a fluid which has a density lower than the density of the medium.

In some embodiments, the cells of interest attached to the low-density body have a density 5% lower than the density of the medium. In other words, the cell of interest and the low-density body to which it is attached form a cell complex and the density of said cell complex is 5% lower than the density of the medium.

In some embodiments, the density is ranging from 0.001 g / cm$^3$ to 1.3 g / cm$^3$.

In the present application, the density of a substance is the ratio of the mass per unit of volume of said substance and the mass per unit of volume of water. Since the mass per unit of volume of water is 1 g/cm$^3$, the density of a substance may, in the present application, be assimilated to the mass per unit of volume of said substance.

Such a differentiation vector is able of lowering the apparent density of the cell it is attached to. Indeed, thanks to the low-density body of the differentiation vector, the density of the differentiation vector is low, compared to the density of a cell and the density of the medium. Thus, the complex formed of a differentiation vector and a cell presents a density which is lower than a density of a lone cell, without any differentiation vector attached to it.

Such differentiation vectors improve the difference in properties between targeted cells of interest attached to said differentiation vectors and other cells, thus improving the efficiency of the segregating.

In some embodiments, the anchor comprises an antibody.

Antibodies are efficient means to selectively bound specific targets, especially when targets are cells. Thus, using an antibody as an anchor improves the selectivity of the method and consequently improve the efficiency of the segregating.

In some embodiments, the anchor comprises a peptide.

In some embodiments, the low-density body comprises oil microdroplets, preferably vegetable oil microdroplets.

Including oil microdroplets in the low-density body allows securing an appropriate density while ensuring the stability of the anchor.

In some embodiments, the low-density body comprises soy bean oil, squalene oil, mineral oil or paraffin microdroplets.

In some embodiments, the low-density body comprises gas bubbles.

Including gas bubbles in the low-density body allows securing an appropriate density while ensuring the stability of the anchor.

In some embodiments, the low-density body comprises carbon tetrafluoride bubbles.

The low-density body may also comprise air and other fluorocarbon gases.

In some embodiments, a typical dimension of the low-density body is greater than 0,5 $\mu$m. In a preferred configuration, said typical dimension is ranging from 1 $\mu$m to 20 $\mu$m, more preferably from 1 $\mu$m to 16 $\mu$m.

In the present application the typical dimension of a component is the length of said component along its largest dimension. For example, the typical dimension can be the diameter of said component.

According to this embodiment, the complex formed by the association of a cell of interest with a differentiation vector has different properties than another cell and the medium. For instance such a complex may present a density at least 5% lower than the density of the medium. Thus, a differentiation vector according to this embodiment improve the efficiency of segregating the cell of interest from the suspension of cells. Moreover, the larger the low-density body is, the larger is the difference in density between the complex differentiation vector/cell of interest and the medium. Accordingly, the efficiency of the method is improved.

In some embodiments, the low-density body is coated with a phospholipidic membrane.

For instance, the phospholipidic membrane may encapsulate a mixture of perfluorobutane and nitrogen gas.

Such a phospholipidic membrane provides an advantageous mean to encapsulate the low-density element, for instance oil or gas, of the low-density body. In particular, it improves the stability of the low-density body.

In some embodiments, at least one cell of the cells of interest is attached to a plurality of differentiation vectors.

When multiple differentiation vectors are attached to a cell of interest, the difference in properties between said cell of interest and the other cells is improved. Therefore, the segregation of cells of interest is easier.

In some embodiments, the differentiation vector is biocompatible. "Biocompatible" refers to the property of a biomaterial to elicit little or no immune response in a given organism, or to be able to integrate with a particular tissue.

In the present disclosure, a biocompatible object is an object that may be injected in a living body, for instance the human body, without creating any undue damages to said body. For instance, a biocompatible object may be injectable into a living body. In other words, a biocompatible object may perform an appropriate response when injected in a living body.

Providing biocompatible differentiation vectors enhances the biocompatibility of the complex cell/differentiation vector. Thus, the cells that are isolated by the segregating may become directly injectable into a patient. In such a case, there is no need to remove the differentiation vector before the injection. Accordingly, considering therapeutic application, it represents an economy of time and of costs. In addition, since no further operation is required before injection, the risk to damage the cells of interest is diminished.

Other differentiation vectors, comprising for instance magnetic beads or PVA (polyvinyl alcohol) bubbles, may be used. PVA vectors are not biocompatible and must be removed before any injection in a living body. However, such a separation is time consuming as it requires washing steps that may induce lose or damage some cells. Thus, biocompatible differentiation vectors are preferable.

In some embodiments, the injection of the suspension operates at a flow rate greater than 0,5 mL/min, preferably greater than 1 mL/min.

Such a flow rate allows segregating a larger number of cells, thus improving the efficiency of the segregating. Also, it allows avoiding the disadvantages of microfluidic systems.

In some embodiments, the acoustic waves are generated at a frequency ranging from 0.1 MHz to 4 MHz.

Such ranges are optimal for resonance with the chamber. Thus, the efficiency of the segregating is improved.

In some embodiments, the suspension evacuated from the first central outlet comprises at least 75% less cells of interest than the suspension provided at the inlet.

The present invention also relates to an acoustophoresis device for performing the method of any one the abovementioned embodiments comprising:

(S1). a chamber configured to be associated with at least one acoustic wave generator for generating acoustic waves within the chamber;

(S2). at least one acoustic wave generator configured to generate acoustic waves within the chamber;

(S3). an inlet (121) in fluid communication with the chamber (11);

(S4). at least three outlets (131, 132, 133) in fluid communication with the chamber (11), said outlets (131, 132, 133) comprising a first central outlet (131) for evacuation of a suspension depleted of cells of interest and at least two second peripheral outlets (132, 133) for evacuation of a suspension comprising cells of interest.

Such a device, used according to the method of the invention, provides the abovementioned advantages.

**FIGURES**

The annexed figures are schematic and only aims to illustrate the present disclosure.

Figure 1 is a flow chart showing steps of a method according to one embodiment of the invention.

Figure 2 is a schematic representation of a differentiation vector according to a first embodiment.

Figure 3 is a schematic representation of a differentiation vector according to a second embodiment.

Figure 4 is a schematic representation of the application of an acoustic field on particles within a chamber.

Figure 5 is a schematic representation of an acoustophoresis device according to an embodiment, wherein cells of interest and other cells flow within the chamber.

Figure 6 is a schematic representation of an acoustophoresis device according to another embodiment, wherein cells of interest and other cells flow within the chamber.

**DETAILED DESCRIPTION**

Examples of embodiments of the invention are hereby described. It is reminded that the invention is not limited to these examples.

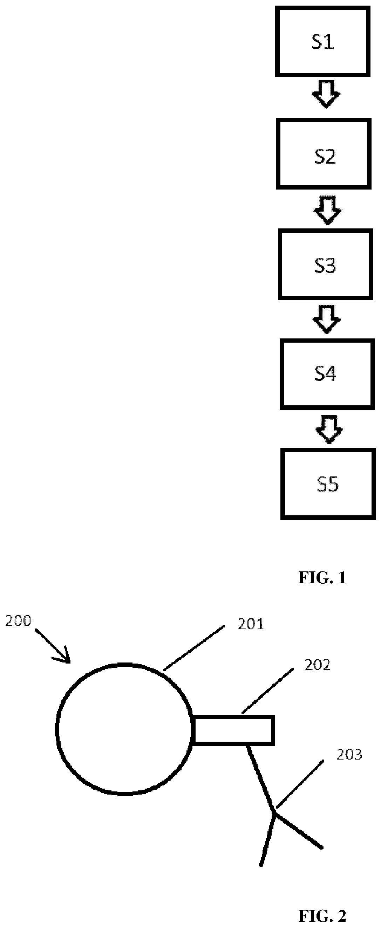

Figure 1 is a flow chart showing steps of a method according to one embodiment of the invention. The method comprises:

(S1). providing an acoustophoresis device 1 which will be described hereafter;

(S2). preparing a suspension comprising cells of interest, wherein cells of interest 2 are attached to a differentiation vector 200, 210;

(S3). injecting the suspension comprising the cells of interest into the acoustophoresis device 1;

(S4). applying an acoustic field by generating acoustic waves inside the acoustophoresis device 1;

(S5). collecting the cells of interest attached to the differentiation vector throughout an outlet of the acoustophoresis device 1.

Cells of interest 2 may be cells that are valuable for the operator or cells that should be excluded from the suspension of cells 3. The cells of the suspension of cells 3 may be same or different type. For example, the method may segregate specific lymphocytes from a suspension of a plurality of lymphocytes, or segregate lymphocytes from a suspension comprising lymphocytes and red blood cells.

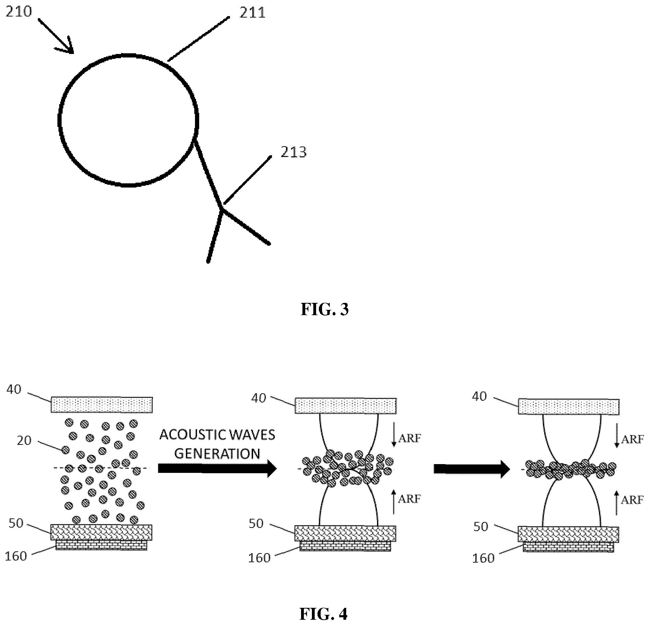

During the preparation of the suspension, a mix of cells of interest 2 and other cells 4 is treated with a solution comprising differentiation vectors 200, 210. Figures 2 and 3 illustrate a differentiation vector according to respectively a first and a second embodiment.

As shown in figure 2, the differentiation vector 200 according to the first embodiment comprises a low-density body 201, a support 202 attached to the low-density body 201 and an antibody 203 attached to the support 202. The support 202 and the antibody 203, considered together, forms an anchor configured to be attached to a cell of interest 2. Indeed, the antibody 203 is configured to selectively bound with the cells of interest 2.

The support 202 may, for example, be streptavidin. On the other hand, the antibody 203 may, for example, be coupled to biotin. In such an example, the biotin of the antibody 203 bound to the support 202 in a non-covalent bounding. The support 202 may also be a peptide or a monoclonal antibody.

As shown in figure 3, the differentiation vector 210 according to the second embodiment comprises a low-density body 211, and an antibody 213. In this embodiment, the antibody 213 is the anchor, which is configured to be attached to a cell of interest 2. Indeed, the antibody 213 is configured to selectively bound with a cell of interest 2.

In both embodiments, the low-density body 201, 211 comprises a fluid which has a density at least 5% lower than the medium. In other words, the low-density body 201, 211 comprises a fluid which has a lower density than the density of water. More generally, the low-density body 201, 211 is configured to present a lower density than the density of the medium carrying the suspension of cells 3. The fluid may be preferably gas bubbles, for instance carbon tetrafluoride bubbles. This fluid may also be oil microdroplets, preferably vegetable oil microdroplets. Further, the low-density body 201, 211 may be coated with a phospholipidic membrane.

Since the density of the low-density body 210, 211 is 5% lower than medium density, the density of the cell of interest attached to the differentiation vector 200, 201 is lower than the density of another cell which is not attached to any differentiation vector 200, 201 and lower than the density of the medium carrying the suspension of cells 3. In other words, the differentiation vector 200, 201 forms a complex with the cell of interest, said complex presenting different properties than the properties of another cell without any differentiation vector 200, 201 attached to it. Multiple differentiation vectors may be attached to a single cell of interest, thus increasing the modification of properties.

The low-density body 210, 211 may present any shape. For instance, the low-density body 210, 211 may be spherical. The typical dimension of the low-density body is greater than 0.5 $\mu$m, preferably greater than 1 $\mu$m, and lesser than 20 $\mu$m.

The low-density body 201, 211 may be biocompatible, such that the differentiation vector 200, 210 may be biocompatible.

When an acoustic field is applied to the suspension of cells, the cells of interest 2 are segregated. The following paragraphs aim to explain the phenomenon leading to said segregation.

As shown in Figure 4, the application of an acoustic field on particles 20 within a chamber between an acoustic wave generator 160 and a reflector 40 induces a movement of said particles 20, allowing to gather said particles 20 at a node of said acoustic field.

In this example, ultrasonic waves are generated in a chamber between a reflector 40 and a wall 50 associated with an acoustic wave generator 160 coupled with a transmitter layer. This enables the creation of an acoustic pressure node in the center of the chamber (depending on the chosen frequency at which the acoustic wave generator 160 operates) and therefore of acoustic radiation forces (ARF). The ARF push the particles 20 towards the pressure node with a force of up to a hundred times gravity equivalent. The particles 20 suspended in the fluid will then migrate to the sound low pressure node (also named acoustic node) and can then remain trapped in this position.

A plurality of acoustic nodes can be created in the chamber, said acoustic nodes can be located at the center of the chamber, or off the center of the chamber. In such a case, a flow of particles 20 passing through the acoustic field will be gathered along one of the acoustic nodes, depending on their properties. For instance, the particles 20 may be gathered along the acoustic nodes of the acoustic field depending on their density.

Indeed, the acoustic force applying on particles 20 is as follows:

$$Facoustic = 4\pi r^3 \psi(\beta, \rho) k_0 E_0 (2 k_0 z)$$

and

$$and \ \psi(\beta, \rho) = \frac{\rho_p + \frac{2}{3}(\rho_p - \rho_0)}{2\rho_p + \rho_0} - \frac{\beta_p}{3\beta_0}$$

Where $\rho_p$ is the density of the particles 20 and $\rho_0$ is the density of the medium, $\beta_p$ is the compressibility of the particles and $\beta_0$ is the compressibility of the medium, r is the radius of the particle 20. $E_0$ and $K_0$ are constant, and $E_0$ may be adjusted by the operator. It appears that the difference in density is preponderant for determining if the particles 20 migrate toward the center of the chamber or toward the periphery of said chamber.

This is particularly advantageous as it allows the isolation of particles 20 within a fluid without any mechanical force, filtration, or centrifugation steps that could damage said particles 20, especially if said particles 20 are fragile like cells.

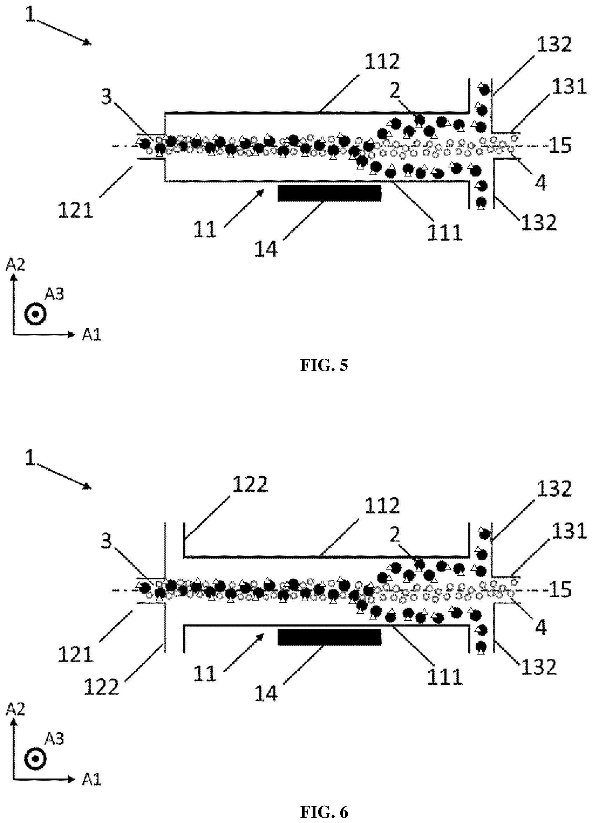

Figure 5 is a schematic representation of an acoustophoresis device 1 according to one embodiment of the invention, configured to perform the method of the invention. The acoustophoresis device 1 comprises:

- a chamber 11 extending along a longitudinal axis A1;

- an acoustic wave generator 14 longitudinally aligned with the longitudinal axis A1 of the chamber 11, and associated with the chamber 11 for generating acoustic waves within the chamber 11;

- an inlet 121 in fluid communication with the chamber 11, said inlet 121 being configured to introduce a suspension of cells 3 in the chamber 1, wherein some cells of the suspension of cells are cells of interest 2 attached to a differentiation marker 200, 210;

- three outlets 131, 132 in fluid communication with the chamber 1, said outlets 131, 132 comprising a first outlet 131 for collecting a suspension comprising cells which are not attached to a differentiation marker and two second outlets for collecting a suspension of cells of interest, which are attached to a differentiation marker.

In the acoustophoresis device 1 of figure 5, a flow of cells of interest 3 and other cells 4 flow within the chamber 11.

The acoustic wave generator 14 is configured to emit an acoustic wave, said acoustic wave having a defined amplitude and a defined frequency, said acoustic wave generator 14 being located between the first inlet 121 and the first outlet 131.

The acoustic wave generator 14 may be a piezoelectric transducer. The transducer may be made of a ceramic, such as lead zirconate, titanate, potassium niobate, or sodium tungstate; a crystal, such as quartz, tourmaline, or gallium phosphate; III-V or II-VI semiconductors such as gallium nitride or zinc oxide; polymers such as polyvinylidene fluoride, polyvinylidene chloride, or polyimide; or a mixture thereof.

The acoustic wave generator 14 may be a longitudinal resonator which allows for an efficient and easy to implement temporary coupling between the acoustic wave generator 14 and the chamber 11.

The power applied to the acoustic wave generator 14 is ranging from 0.2 W to 50 W, preferably from 0.2 W to 25 W, more preferably from 0.5 W to 10 W, even more preferably from 1 W to 4 W.

The chamber extends along a longitudinal axis A1, has a first transverse axis A2 and a second transverse axis A3 perpendicular to the first transverse axis A2, the width being greater than or equal to the thickness, the chamber having first and second walls along the second transverse axis. The length of the chamber is measured along the longitudinal axis A1, the width is measured along the first transverse axis A2 and the thickness is measured along the second transverse axis A3. The chamber comprises a transfer wall 111 coupled with the acoustic wave generator 14 and an opposite wall 112 facing the transfer wall.

The acoustic wave generator 14 is coupled longitudinally to walls 111, 112 of the chamber, in particular to a transfer wall 111. In other words, the acoustic wave generator 14 extends along the same longitudinal axis as the chamber 11.

The acoustic wave generator 14 may be temporarily coupled to the chamber 11. Temporary coupled means removably coupled, such that the acoustic wave generator 14 may be detached from the chamber 11. Such a configuration is particularly advantageous as it allows using a disposable chamber 11 with a non-disposable acoustic wave generator 14, thus ensuring the sterility of the chamber 11, which remains a primordial point in the handling of biological objects.

In a preferred configuration of this embodiment, the temporary coupling is provided by the use of a coupling layer between a wall 111 of the chamber and the acoustic wave generator 14. This advantageously prevents the presence of air bubbles between the chamber 11 and the acoustic wave generator 14, ensuring an improved contact between the chamber 11 and the acoustic wave generator 14. In other words, the coupling layer allows the uninterrupted propagation of acoustic waves between the wave generator 14 and the wall 111. The coupling layer may be a liquid, a gel or any type of thin layer allowing the propagation of acoustic waves. The coupling layer allows for a much more efficient transmission of the acoustic energy between the acoustic wave generator and the chamber.

In this embodiment, the acoustophoresis device 1 comprises an inlet 121 and three outlets 131, 132. The longitudinal axis of the first inlet 121 and the first outlet 131 are aligned with the longitudinal axis A1 of the chamber 11 and with the focusing plane 15 located at the center of the chamber 11, i.e. at about mid-height of said chamber 11. Said axis are perpendicular to the longitudinal axis A1 of the chamber 11, i.e. aligned with the first transverse axis A2. Furthermore, said second outlets 132 are each located on either side of longitudinal axis A1 of the chamber. In other words, second outlets 132 are located opposite from each other according to the longitudinal axis A1.

In some embodiments, illustrated in figure 6, the acoustophoresis device 1 may comprise secondary inlets 122 in fluid communication with the chamber 11. In such a case, the longitudinal axis of the second inlets 122 and the second outlets 132 are aligned, and said second inlets 122 are each located on either side of longitudinal axis A1 of the chamber. In other words, said second inlets 122 are located opposite from each other according to the longitudinal axis A1.

The chamber 11 can be divided into three main zones: the injection zone comprising the inlet 121, the active zone where the acoustic waves are generated and the exit zone comprising the outlets 131, 132. The active zone is designed to optimize the acoustic efficiency in a controlled way through the choice of materials, the thickness of the layers and the width of the chamber 11. The active zone length ranges from 1 cm to 20 cm, preferably from 1 cm to 10 cm, more preferably from 1 cm to 5 cm.

Further, the chamber 11 has a width ranging from 1 mm to 50 mm, preferably from 5 mm to 20 mm, more preferably from 5 mm to 15 mm, said width being the diameter in case of the chamber 11 is a cylinder. Also, the transfer wall 111 and the opposite wall 112 of the chamber 11 has a thickness ranging from 150 $\mu$m to 2 mm, preferably from 300 $\mu$m to 1,5 mm. Otherwise, the transfer wall 111 and the opposite wall 112 are spaced apart by 200 $\mu$m to 400 $\mu$m, preferably 240 $\mu$m to 290 $\mu$m.

Said dimensions allows providing a chamber 11 which is not a microfluidic channel. In other words, the acoustophoresis device 1 is not a microfluidic device. The use of a non-microfluidic channel allows to significantly and easily increase the flow rate in the chamber, and so reduce the processing time for a given volume, while keeping the shear rate low enough for the particles not to be damaged during the process, therefore allowing for the use of this device to produce cell therapy treatments.

Consequently, the flow rate inside the chamber 11 ranges, for a laminar flow rate, from 0.5 mL/min to 100 mL/min. This invention, thanks to differentiation vectors 200, 201, may allow segregating a very small number of rare cells dispersed in a large volume, in a short period of time. In other words, the method of the invention is sensible enough to segregate few cells in a large volume, for instance few hundred cells in a suspension of few hundreds of millilitres, in less than 2 hours. Accordingly, the method is advantageous in terms of costs, time sensitivity and in terms of viability of the cells.

Moreover, the frequency of the acoustic waves generated by the wave generator ranges from 0.1 MHz to 4 MHz.

Further, the transfer wall 111 and the opposite wall 112 of the chamber 11 may be stainless steel, or may comprise a biocompatible material and/or be biocompatible materials, for instance PMMA (polymethylmethylacrylate). On the other hand, the acoustic wave generator 14 may be disposed outside of said transfer wall 111 and opposite wall 112. In such a configuration, the acoustic wave generator 14 may be easily changed.

The suspension of cell 3 is injected at the first inlet 121 with a laminar flow, thus forming a layer of fluid flowing through the chamber 11 without turbulence. Upon application of an acoustic field generated by the acoustic wave generator 14, the cells of interest 2 are deflected from their initial suspensions to the peripheral layer of fluid, as the ARF push the cells of interest 2 towards the radial extremities of the chamber 11 with a force larger than the combined effect of fluid drag force and gravitational force.

Due to their respective density, only the cells of interest 2 are deflected while the flow of the other cells 4 remains unperturbed. Thus, the cells of interest are collected in the second outlets 132, while the other cells 4 are collected at the first outlet 131. According to the method of the invention, the suspension evacuated from the first central outlet 131 comprises at least 50% less, preferably 75% less, cells of interest than the suspension provided at the inlet 121. Moreover, the suspension evacuated from the second outlets 132 comprises at least 80% of cells of interest.

In other examples, the segregation may concern rare cells, for instance cells of a disease such as cancer cells. Such cells may be present in a blood sample or in urine. Thus, these cancer cells are specifically tagged by an adapted differentiation vector 200, 201, so the complex cancer cell/differentiation vector may be segregated by the method of the invention.

The present invention has been described in view of examples of specific embodiments. Modifications may be provided without departing from the scope of the present invention, as defined by the claims. Particularly, individual features of a specific embodiment may be combined to the other embodiments. Also, the description and the drawings should be interpreted as illustrative rather than restrictive.

Moreover, it is clear that features respectively relating to a device or a process are transposable to a process or a device.

**EXAMPLES**

The present invention is further illustrated by the following examples.

Example 1: Positive segregation of cells in a suspension

Positive cell segregation targets the fraction of cells expressing an antigen specific to the antibody of the differentiation vector. The cells of interest will therefore be attached to the adapted differentiation vector 200, 201.

Selection of cells expressing CD34 protein (CD34+) from PBMC (peripheral blood mononuclear cell)

- PBMC are cultured in a suitable culture medium.

- PBMC are washed 2 times from the culture medium with PBS (phosphate buffered saline) by centrifugation and resuspended in PBS.

- Once washed, PBMC are resuspended in PBS.

- The differentiation vectors, in this case 16 μm diameter soy bean oil droplets with phospholipidic membrane, are pre-coupled with CD34 antibodies.

- PBMC cells are mixed with the differentiation vector previously coupled with CD34 antibodies. Mixing is carried out at room temperature for 1 h.

- The mixture is then introduced in the acoustic system, at 1 ml/min.

The cells of interest 2 (CD34+) are the cells bound to the differentiation vector 200, 201. Under acoustophoresis at 2 watts of acoustic power, the cells of interest 2 are pushed towards the second outlets 132 of the chamber 11. The cells to be eliminated that are not attached to the differentiation vector are pushed towards the first outlet 131 of the chamber 11. The inventors have observed that more than 75 % of the cells of interest 2 move towards the second outlets 132, while 80 % of said cells moving towards the second outlets 132 are target cells 2. In other words, the purity of the flow of cells outputted from the second outlets is 80%. This method has the advantage that the purity is high.

Example 2: Negative segregation of cells in a suspension

Negative cell segregation targets the fraction of cells not expressing specific antigen to the antibody of the differentiation vector. The selected cells are the cells which are indifferent to any interaction with the antibody of the differentiation vector. The cells of interest will not be bound to the differentiation vectors.

Selection of cells that are not expressing CD34 protein (CD34-) from PBMC

- PBMC are cultured in a suitable culture medium.

- PBMC are washed 2 times from the culture medium with PBS by centrifugation and resuspended in PBS.

- Once washed, PBMC are resuspended in PBS.

- The differentiation vectors are pre-coupled with CD3, CD8, CD4 antibodies.

- The cells are mixed with the differentiation vector previously coupled with CD3, CD8, CD4 antibodies. Mixing was carried out at room temperature for 1 h.

- The mixture was then run over the acoustic system.

The cells of interest 2 (CD34-) are the cells which are free any differentiation vectors 200, 201. The cells attached to the differentiation vector 200,201 are, under acoustophoresis at 2 watts of acoustic power, pushed towards the second outlets 132 of the chamber 1 and are eliminated, whereas the cells of interest 2 are pushed towards the first outlet 131 of the chamber 11. The inventors have observed that more than 75 % of the cells to be eliminated move to the second outlets 132, while 80 % of the cells moving towards the first outlet 131 are target cells 2. In other words, the purity of the flow of cells outputted from the first outlet 131 is 80%. This method has the advantage that no vector is attached to the cells of interest.

**REFERENCES**

1: acoustophoresis device
2: cells of interest
3: suspension of cells
4: other cells
11: chamber
14: acoustic wave generator
15: focusing plane
20: particles
40: reflector
50: wall
111: transfer wall
112: opposite wall
121: inlet
122: secondary inlets
131: first outlet
132: second outlets
160: acoustic wave generator
200, 210: differentiation vector
201, 211: low-density body
202: support
203, 213: antibody

**Claims**

1. A method for segregating cells (2) in a suspension comprising:

a. providing an acoustophoresis device (1) comprising:

i. a chamber (11) configured to be associated with at least one acoustic wave generator (14) for generating acoustic waves within the chamber (11);
ii. at least one acoustic wave generator (14) configured to generate acoustic waves within the chamber (11);
iii. at least one inlet (121) in fluid communication with the chamber (11);
iv. at least three outlets (131, 132) in fluid communication with the chamber (11), said outlets (131, 132) comprising a first central outlet (131) for evacuation of a first part of the suspension and at least two second peripheral outlets (132) for evacuation of a second part of the suspension;

b. preparing a suspension comprising cells of interest in a medium, wherein cells of interest are attached to a differentiation vector (200, 210),
c. injecting the suspension comprising the cells of interest into the chamber (11) at the inlet (121);
d. applying an acoustic field by generating acoustic waves inside the chamber (11);
e. collecting the cells of interest attached to the differentiation vector (200, 210) throughout the at least two second peripheral outlets (132).

2. The method of claim **1,** wherein the differentiation vector (200, 210) comprises:

a. an anchor configured to be attached to a cell of interest and
b. a low-density body (201, 211) attached to the anchor,

wherein the low-density body (201, 211) comprises a fluid which has a density lower than the density of the medium.

3. The method of claim **1** or **2,** wherein the second part of the suspension comprises cells of interest, while the first part of the suspension is depleted of cells of interest.

4. The method of claim **2** or **3,** wherein the cells of interest attached to the low-density body (201, 211) have a density at least 5% lower than the medium.

5. The method of claim **4,** wherein the density is ranging from 0.001 g / cm$^3$ to 1.3 g / cm$^3$.

6. The method of any of claim **2** to **5,** wherein the anchor comprises an antibody (203, 213) or a peptide.

7. The method of any of claims **2** to **6,** wherein the low-density body (201, 211) comprises oil microdroplets, preferably vegetable oil microdroplets or mineral oil microdroplets.

8. The method of any of claims **2** or **6,** wherein the low-density body (201, 211) comprises gas bubbles, preferably carbon tetrafluoride bubbles.

9. The method of any one of claims **1** to **8,** wherein a typical dimension of the low-density body (201, 211) is greater than 0,5 $\mu$m.

10. The method of any one of claims **1** to **9,** wherein the low-density body (201, 211) is coated with a phospholipidic membrane.

11. The method of any one of claims **1** to **10,** wherein at least one cell of the cells of interest is attached to a plurality of differentiation vectors (200, 210).

12. The method of any one of claims **1** to **11,** wherein the differentiation vector (200, 210) is biocompatible.

13. The method according to any one of claims **1** to **12,** wherein the injection of the suspension operates at a flow rate greater than 0.5 mL/min.

14. The method according to any one of claims **1** to **13,** wherein the acoustic waves are generated at a frequency ranging from 0.1 MHz to 4 MHz.

15. An acoustophoresis device (1) for performing the method of any one of claims 1 to **14** comprising:

   a. a chamber (11) configured to be associated with at least one acoustic wave generator (14) for generating acoustic waves within the chamber (11);
   b. at least one acoustic wave generator (14) configured to generate acoustic waves within the chamber (11);
   c. an inlet (121) in fluid communication with the chamber (11);
   d. at least three outlets (131, 132) in fluid communication with the chamber (11), said outlets (131, 132) comprising a first central outlet (131) for evacuation of a first part of the suspension and at least two second peripheral outlets (132) for evacuation of a second part of the suspension.,

**FIG. 1**

**FIG. 2**

**FIG. 3**

**FIG. 4**

FIG. 5

FIG. 6

Europäisches
Patentamt

European
Patent Office

Office européen
des brevets

**EUROPEAN SEARCH REPORT**

Application Number

EP 24 30 5374

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/290829 A1 (FIERING JASON O [US] ET AL) 26 September 2019 (2019-09-26) * the whole document * | 1-15 | INV. C12M1/42 C12M1/00 |
| X | US 2023/173489 A1 (WU DI [US] ET AL) 8 June 2023 (2023-06-08) * figures 6A-6C * * paragraph [0003] - paragraph [0022] * * paragraphs [0061], [0075], [0076] * * paragraph [0099] - paragraph [0116] * * claims 1-20 * | 1-15 | |
| X | WO 2022/187319 A2 (UNIV LOUISVILLE RES FOUND INC [US]) 9 September 2022 (2022-09-09) * page 2 - page 8 * * figures 1A-1E, 2, 6 * * claims 1-35 * | 1-3,8, 10,12, 14,15 | |
| A | US 2016/355776 A1 (LIPKENS BART [US] ET AL) 8 December 2016 (2016-12-08) * paragraph [0007] - paragraph [0019] * * claims 1-20; figure 6 * | 1-15 | TECHNICAL FIELDS SEARCHED (IPC) C12M |

The present search report has been drawn up for all claims

| Place of search | Date of completion of the search | Examiner |
|---|---|---|
| The Hague | 11 September 2024 | González Ferreiro, M |

EPO FORM 1503 03.82 (P04C01)

## ANNEX TO THE EUROPEAN SEARCH REPORT
## ON EUROPEAN PATENT APPLICATION NO.

EP 24 30 5374

This annex lists the patent family members relating to the patent documents cited in  the above-mentioned European search report.
The members are as contained in the European Patent Office EDP file on
The European Patent Office is in no way liable for these particulars which are merely given for the purpose of information.

11-09-2024

| Patent document cited in search report | | | Publication date | Patent family member(s) | | Publication date |
|---|---|---|---|---|---|---|
| US  2019290829 | A1 | 26-09-2019 | | CA | 3027691 A1 | 01-02-2018 |
| | | | | EP | 3490712 A1 | 05-06-2019 |
| | | | | ES | 2908736 T3 | 03-05-2022 |
| | | | | JP | 7340642 B2 | 07-09-2023 |
| | | | | JP | 2019527536 A | 03-10-2019 |
| | | | | JP | 2022084801 A | 07-06-2022 |
| | | | | US | 2019290829 A1 | 26-09-2019 |
| | | | | WO | 2018022158 A1 | 01-02-2018 |
| US  2023173489 | A1 | 08-06-2023 | | US | 2023173489 A1 | 08-06-2023 |
| | | | | WO | 2023102474 A1 | 08-06-2023 |
| WO 2022187319 | A2 | 09-09-2022 | | NONE | | |
| US  2016355776 | A1 | 08-12-2016 | | US | 2016355776 A1 | 08-12-2016 |
| | | | | US | 2017159007 A1 | 08-06-2017 |
| | | | | US | 2017204360 A1 | 20-07-2017 |

EPO FORM P0459

For more details about this annex : see Official Journal of the European Patent Office, No. 12/82